# EUROPÄISCHE PATENTANMELDUNG

(11) **EP 3 581 916 A1**
(43) Veröffentlichungstag der Anmeldung: **18.12.2019**
(21) Anmeldenummer: 19178763.9
(22) Anmeldetag: 06.06.2019
(51) Int. Cl.: G01N 15/06, G01N 27/74, G01N 33/543

(54) **VORRICHTUNG UND VERFAHREN ZUR MAGNETISCHEN PARTIKELBESTIMMUNG**

(30) Priorität: 15.06.2018 DE 102018209651
(71) Anmelder: Forschungszentrum Jülich GmbH, 52425 Jülich (DE)
(72) Erfinder: Achtsnicht, Stefan, 52349 Düren (DE); Krause, Prof. Dr., Hans-Joachim, 52499 Baesweiler (DE)
(74) Vertreter: Gille Hrabal

(57) **Zusammenfassung**

Die Erfindung betrifft eine Vorrichtung zur Bestimmung von magnetischen Partikeln umfassend einen Probenhalter für magnetische Partikel, zwei Magnetfelderzeugungseinrichtungen (1, 4; 2, 5) für ein Anlegen eines magnetischen Niederfrequenzwechselfeldes (f2) und eines magnetischen Hochfrequenzwechselfeldes (f1) an magnetische Partikel, die durch den Probenhalter gehalten werden können, eine Messeinrichtung, mit der eine Phasenverschiebung zwischen zumindest einer Phase eines angelegten magnetischen Wechselfelds (f1, f2) und einer Phase eines Antwortmagnetfelds (f3) ermittelt werden kann und/oder ein Verhältnis von Harmonischen, die das Antwortmagnetfeld (f3) umfasst, ermittelt werden kann, eine Auswerteeinrichtung, mit der aus einer ermittelten Phasenverschiebung und/oder aus einem ermittelten Verhältnis von Harmonischen die Art und Charakteristiken von magnetischen Partikel bestimmt werden kann.

Die Erfindung betrifft außerdem ein Verfahren zur Bestimmung und Charakterisierung von magnetischen Partikeln mit der Vorrichtung.

## Beschreibung

Die vorliegende Erfindung betrifft ein Verfahren und eine Vorrichtung zur Bestimmung von magnetischen Partikeln. Die zu bestimmenden magnetischen Partikel sind regelmäßig magnetische Nanopartikel mit Durchmessern von weniger als 1000 Nanometern, vorzugsweise von weniger als 500 nm. Durchmesser von Nanopartikeln liegen typischerweise zwischen 1 bis 100 Nanometern.

Magnetische Partikel zu bestimmen meint, das Vorhandensein von magnetischen Partikeln in einer Probe nachzuweisen, die Menge der magnetischen Partikel zu ermitteln und/oder die Art der magnetischen Partikeln zu ermitteln.

Werden Antikörper oder Aptamere an die Oberfläche von magnetischen Partikeln wie zum Beispiel magnetische Nanopartikel gebunden, können sie zur spezifischen Bindung an biologische Zielsubstanzen vorbereitet werden. Die magnetischen Partikel können dann als Marker für die Anwesenheit von Zielsubstanzen dienen. Die magnetischen Partikel, die mit biologischen Substanzen wie z.B. Krankheitserregern, Viren, Bakterien, Proteinen oder Zellen verbunden sind, können zum Beispiel für diagnostische Zwecke bei der Durchführung von Immunoassays verwendet werden, aber auch in der Umweltwissenschaft, in der Lebensmittelkontrolle und in der Medizin. Es ist also bekannt, magnetische Partikel als Marker für Biomoleküle und biologische Zielsubstanzen einzusetzen. Es besteht daher Bedarf, magnetische Partikel zu detektieren und zu quantifizieren.

Ein Verfahren und eine Vorrichtung, um superparamagnetische und/oder ferromagnetische Partikel zu detektieren und zu quantifizieren, sind aus der Patentanmeldung US 2007/0155024 A1 bekannt.

Ferromagnetische Werkstoffe magnetisieren sich in einem externen Magnetfeld (H) so, dass sich die magnetische Flussdichte (B), auch magnetische Induktion genannt, in ihrem Inneren im Vergleich zum Außenraum erhöht. Der Faktor der Flussdichteerhöhung im Vergleich zum leeren Raum wird durch die magnetische Permeabilität (µᵣ) oder die magnetische Suszeptibilität (µᵣ - 1) des Werkstoffs beschrieben. Bei einem ferromagnetischen Werkstoff ist µᵣ >> 1. Die magnetische Induktion hängt in nicht linearer Weise von dem externen Magnetfeld ab. Dieser Zusammenhang wird auch nichtlineare Magnetisierung oder nichtlineare Magnetisierungseigenschaft genannt.

Superparamagnetismus bezeichnet die magnetische Eigenschaft sehr kleiner Teilchen eines ferromagnetischen oder ferrimagnetischen Materials, auch bei Temperaturen unterhalb der Curie-Temperatur keine bleibende Magnetisierung zu halten, wenn ein zuvor angelegtes Magnetfeld abgeschaltet wurde. Bei superparamagnetischen Werkstoffen gibt es ebenfalls einen nichtlinearen Zusammenhang zwischen einem angelegten Magnetfeld und der daraus resultierenden magnetischen Flussdichte.

Gemäß der aus der US 2007/0155024 A1 bekannten Lehre werden nichtlineare Magnetisierungseigenschaften ausgenutzt, um mithilfe eines Frequenzmischverfahrens magnetische Partikel zu detektieren und zu quantifizieren. An die in einem Gefäß befindlichen magnetischen Partikel werden zwei magnetische Wechselfelder mit unterschiedlichen Frequenzen angelegt. Das Anlegen der zwei magnetischen Wechselfelder bewirkt eine magnetische Induktion. Das aus diesem Verfahren resultierende Magnetfeld wird gemessen und ausgewertet. So werden bei einer zuvor festgelegten Phasenverschiebung zwischen angelegten magnetischen Wechselfeldern und Antwortmagnetfeldern Amplitudenhöhen bei Antwortmagnetfeldern bestimmt. Aus dem Vorhandensein von entsprechenden Amplituden wird auf das Vorhandensein von magnetischen Partikeln geschlossen. Aus der Höhe einer Amplitude wird auf die Menge von vorhandenen magnetischen Partikeln geschlossen. Es kann so also die Anwesenheit von magnetischen Partikeln detektiert werden. Außerdem kann die Menge von magnetischen Partikeln quantitativ bestimmt werden.

Es ist allerdings nicht möglich, die Art/spezifische Charakteristiken von magnetischen Partikeln zu bestimmen, also beispielweise zu bestimmen, aus welchem Werkstoff detektierte magnetische Partikel bestehen oder welche Größen die magnetischen Kristallite/Kerne der Partikel aufweisen. Die Bestimmung einer Art umfasst auch eine Information über einen Bindungszustand einer Zielsubstanz an magnetische Partikel. Es ist also durch die vorliegende Erfindung auch möglich, solche Bindungszustände zu bestimmen. Nachfolgend wird diese Bestimmung qualitative Bestimmung genannt.

Es ist Aufgabe der Erfindung, den aus der US 2007/0155024 A1 bekannten Stand der Technik weiter zu entwickeln.

Zur Lösung der Aufgabe dient eine Vorrichtung mit den Merkmalen des ersten Anspruchs. Ein Verfahren umfasst zur Lösung der Aufgabe die Merkmale des Nebenanspruchs. Vorteilhafte Ausgestaltungen ergeben sich aus den abhängigen Ansprüchen.

Gemäß der vorliegenden Erfindung werden durch einen Probenhalter gehaltene magnetische Partikel einem ersten magnetischen Wechselfeld (nachfolgend auch Niederfrequenzmagnetfeld genannt) ausgesetzt. Zeitgleich werden die magnetischen Partikel einem zweiten magnetischen Wechselfeld (nachfolgend auch Hochfrequenzmagnetfeld genannt) mit einer höheren Frequenz als das Niederfrequenzmagnetfeld ausgesetzt.

Durch die beiden magnetischen Wechselfelder und die magnetischen Partikel entsteht ein Magnetfeld, welches nachfolgend Antwortmagnetfeld genannt wird. Das Antwortmagnetfeld ist ein magnetisches Wechselfeld, welches in erster Linie von der Art der magnetischen Partikel und ihrer Konzentration abhängt.

Aufgrund der nichtlinearen Magnetisierungseigenschaften der magnetischen Partikel umfasst das Antwortmagnetfeld zusätzliche Frequenzkomponenten, die nachfolgend auch Frequenzmischkomponenten genannt werden.

Das Antwortmagnetfeld umfasst Harmonische. Als Harmonische wird eine harmonische Schwingung bezeichnet, deren Frequenz ein ganzzahliges Vielfaches einer Grundfrequenz ist oder die Summe mehrerer solcher ganzzahligen Vielfache von Frequenzen. Letztere werden auch als Frequenzmischkomponenten bezeichnet. Eine Harmonische oberhalb der Grundfrequenz wird daher auch Oberschwingung oder Oberwelle genannt. So treten gemäß der vorliegenden Erfindung Frequenzmischkomponenten mit Frequenzen auf, die ein ungerades Vielfaches der Frequenz f1 des Hochfrequenzmagnetfeldes sind. Das Antwortmagnetfeld umfasst also Frequenzen, die zum Beispiel das Dreifache oder Fünffache der Frequenz f1 des Hochfrequenzmagnetfeldes betragen, also 3·f1 oder 5.f1.

Das Antwortmagnetfeld umfasst Frequenzmischkomponenten mit Frequenzen, deren Frequenz aus einer ungeraden Anzahl von Bestandteilen der Frequenz f1 des Hochfrequenzmagnetfeldes und der Frequenz f2 des Niederfrequenzmagnetfeldes zusammengesetzt sind. Es können also Harmonische mit Frequenzen resultieren, die f1 + 2.f2, f1 - 2·f2, f1 + 4·f2 oder f1 - 4·f2 betragen.

Der vorliegenden Erfindung liegt die Erkenntnis zugrunde, dass es für die Art der magnetischen Partikel charakteristische Phasenverschiebungen zwischen angelegten magnetischen Wechselfeldern und induziertem Antwortmagnetfeld gibt und/oder charakteristische Phasenverschiebungen zwischen Harmonischen. Der vorliegenden Erfindung liegt außerdem die Erkenntnis zugrunde, dass es für die Art der magnetischen Partikel charakteristische Verhältnisse von Harmonischen im induzierten Antwortmagnetfeld gibt. Die absolute Menge der magnetischen Partikel beeinflusst dagegen weder diese Verhältnisse von Harmonischen noch vorgenannte Phasenverschiebungen. Es wird daher erfindungsgemäß eine Phasenverschiebung zwischen angelegten magnetischen Wechselfeldern und Antwortmagnetfeld ermittelt. Alternativ oder ergänzend wird eine Phasenverschiebung zwischen Harmonischen ermittelt. Alternativ oder ergänzend wird ein Verhältnis von Harmonischen ermittelt. Aus einer ermittelten Phasenverschiebung und/oder aus einem ermittelten Verhältnis wird die Art von magnetischen Partikeln ermittelt. Am einfachsten geschieht dies durch Vergleich mit Referenzwerten. Es sind also zunächst Phasenverschiebungen und/oder Verhältnisse von Harmonischen von bekannten Arten von Magnetpartikeln gemessen worden, um so Referenzwerte zu erhalten. Durch Vergleich von Messwerten mit Referenzwerten können im Fall von Übereinstimmungen Magnetpartikelarten bestimmt werden. Es kann also beispielsweise ein Werkstoff identifiziert werden, aus denen Magnetpartikel einer durch einen Probenhalter gehaltenen Probe bestehen.

Eine Vorrichtung zur qualitativen Bestimmung von magnetischen Partikeln umfasst daher einen Probenhalter für magnetische Partikel und zwei Magnetfelderzeugungseinrichtungen für ein Anlegen von zwei magnetischen Wechselfeldern an magnetische Partikel. Mit zwei Magnetfelderzeugungseinrichtungen ist gemeint, dass einerseits das zuvor beschriebene Niederfrequenzmagnetfeld erzeugt werden kann und andererseits das zuvor beschriebene Hochfrequenzmagnetfeld. Es sind dafür beispielsweise nicht zwingend zwei Spulen erforderlich. Es kann also eine genügen. Zwei Magnetfelderzeugungseinrichtungen liegen also vor, wenn die Vorrichtung so eingerichtet ist, dass zwei magnetische Wechselfelder erzeugt werden können, die sich durch ihre Frequenz unterscheiden. Die Magnetfelderzeugungseinrichtungen sind vorzugsweise so beschaffen, dass zumindest die Frequenz eines jeden Magnetfeldes gewählt bzw. eingestellt werden kann. Vorzugsweise ist die magnetische Feldstärke ebenfalls wählbar bzw. einstellbar.

Durch den Probenhalter können magnetische Partikel gehalten werden. Werden magnetische Partikel durch den Probenhalter gehalten, so liegen die zwei magnetischen Wechselfelder an den gehaltenen magnetischen Partikeln an, wenn die zwei magnetischen Wechselfelder durch die beiden Magnetfelderzeugungseinrichtungen erzeugt werden. Die zwei magnetischen Wechselfelder liegen im Sinne der vorliegenden Erfindung an den durch den Probenhalter gehaltenen magnetischen Partikel an, wenn dadurch ein Antwortmagnetfeld erzeugt werden kann, welches also beispielsweise im Fall von ferromagnetischen Partikeln die zuvor beschriebenen Harmonischen umfasst.

Es ist eine Messeinrichtung vorhanden, mit der eine Phasenverschiebung zwischen zumindest einer Phase eines angelegten magnetischen Wechselfelds und einer Phase eines Antwortmagnetfelds ermittelt werden kann. Alternativ oder ergänzend kann ein Verhältnis von Harmonischen, die das Antwortmagnetfeld umfasst, ermittelt werden.

Es gibt eine Auswerteeinrichtung, mit der aus einer ermittelten Phasenverschiebung und/oder aus einem ermittelten Verhältnis von Harmonischen die Art von magnetischen Partikeln bestimmt werden kann. Im Unterschied zu dem aus der Druckschrift US 2007/0155024 A1 bekannten Stand der Technik können magnetische Partikel also durch die erfindungsgemäße Vorrichtung und das erfindungsgemäße Verfahren qualitativ bestimmt werden. So ist es beispielsweise möglich, superparamagnetische Partikel aus Maghemit (γ-Fe₂O₃) oder Magnetit (Fe₃O₄) mit einem mittleren hydrodynamischen Durchmesser von beispielsweise 500 nm voneinander zu unterscheiden. Vorzugsweise ist die Vorrichtung darüber hinaus so eingerichtet, dass aus Amplitudenhöhen des Antwortmagnetfeldes magnetische Partikel auch quantitativ bestimmt werden können.

Vorzugsweise umfasst die Vorrichtung einen Analog-Digital-Wandler zur gleichzeitigen Erfassung zahlreicher Frequenzmischkomponenten, die aufgrund ihrer nichtlinearen Magnetisierungseigenschaften aus magnetischen Partikeln erzeugt werden. Eine durch den Probenhalter gehaltene Probe, die magnetische Partikel enthält, wird durch das Niederfrequenzmagnetfeld und durch das Hochfrequenzmagnetfeld einer magnetischen Mehrfrequenz-Anregung ausgesetzt. Durch den Analog-Digital-Wandler wird das Antwortmagnetfeld und damit also die magnetische Antwort dieser magnetischen Partikel schnell und präzise aufgezeichnet und digitalisiert. Durch diese Ausgestaltung können magnetische Partikel verbessert bestimmt werden. Die digital aufgezeichnete magnetische Antwort der Partikel wird digital analysiert, beispielsweise hinsichtlich der Amplitude und/oder der Phase der Frequenzmischkomponenten.

In einer Ausgestaltung umfasst die Vorrichtung eine Signalvorverarbeitungseinrichtung, mit der das Antwortmagnetfeld auf einen niedrigeren Frequenzbereich demoduliert wird. Hierdurch gelingt es weiter verbessert, zahlreiche Frequenzmischkomponenten gleichzeitig zu erfassen, um so magnetische Partikel weiter verbessert bestimmen zu können. So haben sich in der Praxis Messungen bewährt, bei denen die Hochfrequenz oder eine etwas kleinere Frequenz, z.B. 1 kHz, als die Hochfrequenz abgezogen wurden.

Die Signalvorverarbeitungseinrichtung umfasst in einer Ausgestaltung ein oder mehrere Frequenzfilter, um geeignete Frequenzbereiche aus dem Antwortmagnetfeld herauszufiltern, um so magnetische Partikel weiter verbessert bestimmen zu können. Verschiedene Filter sind möglich. Eine optimierte Wahl ist abhängig von der späteren Verwendung des Aufbaus. Werden später zur Anregung nur feste Frequenzen f1 und f2 verwendet, so sind Bandstoppfilter bei den Anregefrequenzen f1 und f2 geeignet, um starke (unter Umständen direkt induzierte) Signale der Anregefrequenzen f1 und f2 im Messsignal zu unterdrücken/minimieren. Wird mindestens eine Anregefrequenz f1 oder f2 gescannt, d.h. über einen Messzeitraum variiert, um weitere Informationen über die Partikel zu sammeln, so ist zu bevorzugen, dass keine der entstehenden und zu analysierenden Harmonischen durch diesen Filter geblockt werden. Weiterhin praktisch und in typischer Weise eingesetzte Filter sind Antialiasing-Filter bei der Verwendung eines Analog-Digital-Wandlers, zum Beispiel, um Signale, die frequenzmäßig größer als die größte noch zu messende Harmonische sind, zu unterdrücken, z.B. bei 2·HF. Zusätzlich sind in einer Ausgestaltung Hochpassfilter zum Unterdrücken der Niederfrequenzanregung im Einsatz. Weiterhin kann es je nach Umgebung sinnvoll sein, Netzfrequenzen zu unterdrücken. Bei all diesen Filterwahlen ist vorzugsweise zu beachten, dass diese nur das Ausgangssignal verändern und auf die Proben unter Umständen weiterhin ein Magnetfeld mit der Netzfrequenz einwirkt, was vorzugsweise durch eine automatische Fehlerkontrolle mit berücksichtigt wird. In einer nachfolgenden Signalauswertung wird dieser Peak entsprechend korrigiert.

Die Signalvorverarbeitungseinrichtung umfasst in einer Ausgestaltung ein oder mehrere Verstärker, mit denen das Antwortmagnetfeld verstärkt werden kann, um so magnetische Partikel weiter verbessert bestimmen zu können.

Im Anschluss an Vorverarbeitung durch die Signalvorverarbeitungseinrichtung werden die vorverarbeiteten Messwerte mit dem Analog-Digital-Wandler (ADC) digitalisiert und anschließend weiter verarbeitet.

In einer Ausgestaltung werden die digitalisierten Messwerte, also die digital aufgezeichnete magnetische Antwort der magnetischen Partikel, durch einen Datenträger gespeichert, um zu einem späteren Zeitpunkt mit unterschiedlichen Analysealgorithmen und/oder auf einer anderen, leistungsfähigeren Computerhardware die digital aufgezeichnete magnetische Antwort weiter zu verarbeiten. Insbesondere wird die die digital aufgezeichnete magnetische Antwort bezüglich ihrer Amplituden und/oder Phasen der verschiedenen Harmonischen analysiert, um so magnetische Partikel weiter verbessert zu bestimmen. Zeitgleich oder alternativ kann eine Echtzeit/online-Auswertung/Analyse erfolgen.

Durch die vorgenannten Ausgestaltungen, durch die zahlreiche Frequenzmischkomponenten gleichzeitig erfasst werden können, kann die Messzeit erheblich reduziert werden. Zeitlich aufeinanderfolgende Messungen sind nicht erforderlich. Darüber hinaus ermöglicht die gleichzeitige Erfassung der Frequenzmischkomponenten die Korrektur von Messsignal-Schwankungen, die auf Änderungen der Anregungsfeldamplituden oder externe Störungen zurückzuführen sind, da diese Änderungen auch in allen Frequenzmischkomponenten auftreten, z.B. elektronische Störungen oder magnetische Störfelder.

Werden verschiedene Oberwellen nicht gleichzeitig aufgezeichnet, so können Änderungen an der Probe, die die magnetischen Partikel enthält, z.B. aufgrund von Temperaturschwankungen oder chemischen Reaktionen oder während einer Durchflussmessung zu einer Änderung des magnetischen Antwortsignals mit der Zeit führen. Dies wird durch die zeitgleiche Erfassung vermieden.

Vorzugsweise wird das Antwortmagnetfeld kontinuierlich gemessen. Mit dem Analog-Digital-Wandler wird die Magnetisierung der magnetischen Partikel digital als Funktion der Zeit erfasst und zwar insbesondere mit hoher Auflösung und/oder hoher Abtastrate. Dieses digitalisierte Signal wird numerisch hinsichtlich seiner Harmonischen analysiert.

Unterschiedliche Amplituden und Phasen können digital aus der Messung des Zeitverlaufs des magnetischen Antwortsignals der magnetischen Partikel bestimmt werden. Die Auswertung wird beispielsweise in einer Ausgestaltung der Erfindung durch eine schnelle Fourier-Transformation des abgetasteten Signals durchgeführt. Die Fourier-Transformation kann verwendet werden, um die Amplituden und die Phasen der Antwort bei verschiedenen Frequenzen f1 ± n·f2 zu extrahieren, wobei n irgendeine natürliche ganze Zahl ist, n = 1, 2, 3, 4,

Alternativ können die Amplituden und die Phasen des magnetischen Antwortmagnetfeldes bei verschiedenen Frequenzen f1 ± n·f2 extrahiert werden, indem z.B. eine digitale (Doppel-) Lock-In-Auswertung durchgeführt wird.

Die Signalzeitverfolgung kann nicht nur in Echtzeit analysiert werden, sondern sie kann auch in einer Ausgestaltung der Erfindung aufgezeichnet und in einem Speicher eines Computers gespeichert werden. Numerische Analysen nach einer der oben beschriebenen Verfahren können in einer Ausgestaltung der Erfindung auch nachträglich in einer Offline-Auswertung durchgeführt und/ oder wiederholt werden. Dadurch können die Auswertealgorithmen nach der Messung variiert und optimiert werden.

Unterschiedliche Auswertealgorithmen können in einer Ausgestaltung der Erfindung verglichen und kombiniert werden. Es ist in einer Ausgestaltung der Erfindung möglich, die Signale bzw. Messwerte mit einem einfachen Aufzeichnungssystem aufzuzeichnen und später die Auswertung auf einem anderen leistungsfähigeren Computer durchzuführen. Es ist in einer Ausgestaltung der Erfindung auch möglich, eine Online-Auswertung durchzuführen, beispielsweise mit einem Mikrocontroller oder mit einem Field-Programmable Gate Array (FPGA).

In einer Ausgestaltung der Erfindung umfasst die Vorrichtung eine Auslöseeinrichtung, die in Abhängigkeit von der Phase des Niederfrequenzmagnetfeldes oder des Hochfrequenzmagnetfeldes das Messen durch die Messeinrichtung auslöst, also triggert. Da im Zeitpunkt des Auslösens dann die Phasenlage des Niederfrequenzmagnetfeldes und/oder des Hochfrequenzmagnetfeldes bekannt ist, kann auf technisch einfache Weise eine Phasenverschiebung zwischen dem Niederfrequenzmagnetfeld und dem Antwortmagnetfeld ermittelt werden und/oder zwischen dem Hochfrequenzmagnetfeld und dem Antwortmagnetfeld. Es ist zu bevorzugen, dass in Abhängigkeit von der Phase des Magnetfelds ausgelöst wird, welches die niedrigste Frequenz hat, um auf technisch einfache Weise eine gesuchte Phasenverschiebung ermitteln zu können. Beträgt beispielsweise die Frequenz f2 des Niederfrequenzmagnetfeldes 50 Hz oder 100 Hz und die Frequenz f1 des Hochfrequenzmagnetfeldes 10 kHz oder 100 kHz, dann wird also vorteilhaft in Abhängigkeit von dem Niederfrequenzmagnetfeld durch die Auslöseeinrichtung der Start der Erfassung von Messwerten ausgelöst. Beispielsweise wird im Fall eines ansteigenden Magnetfeldes bei Nulldurchgang die Erfassung der Messwerte ausgelöst, also gestartet.

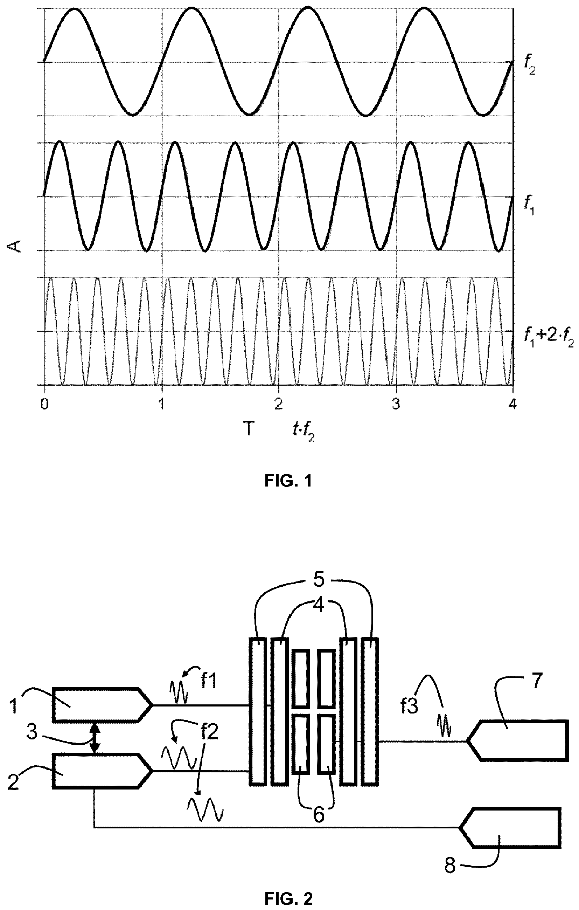

Ist die Frequenz f1 des Hochfrequenzmagnetfelds als ganzzahliges Vielfaches der Frequenz f2 des Niederfrequenzmagnetfelds gewählt, so ist sichergestellt, dass für diese immer die gleiche Phasenbeziehung zu einem spezifischen Zeitpunkt des Niederfrequenzmagnetfelds gilt. Es lässt sich weiterhin zeigen, dass das gleiche dann auch für alle auftretenden Mischfrequenzen gilt. Damit lässt sich auf technisch einfache Weise die Phasenverschiebung der Mischfrequenzen zwischen der detektierten Mischfrequenz und einer virtuellen Mischfrequenz, welche durch Kenntnis der Phasenbeziehung zwischen Nieder- und Hochfrequenz sowie der Phasenlage der Niederfrequenz gewonnen wurde, ermitteln. Durch diese bevorzugte Wahl wird sichergestellt, dass die gleiche Phasenbeziehung aller Signale nach einer vollständigen Periode der niedrigsten Anregungsfrequenz beibehalten wird. Als ein Beispiel zeigt Fig. 1 die Frequenzen f1 (Hochfrequenzerregung), f2 (Niederfrequenzerregung) und f1 + 2·f2 (Mischfrequenzkomponente, die in einer magnetischen Nanopartikelprobe erzeugt wird). Um die Lesbarkeit des Graphen zu verbessern, wurden die Frequenzen sehr ähnlich gewählt. In der Praxis wird bevorzugt der Wert der hohen Frequenz f1 zur Lösung der Aufgabe mindestens 100-mal größer als der Wert der niedrigen Frequenz f2 gewählt.

In einer Ausgestaltung wird zu einem definierten Zeitpunkt der Messung wie zum Beispiel im Startzeitpunkt einer Messung (also der Zeitpunkt des Starts der Erfassung von Messwerten) die Phasenlage des modulierendes Magnetfelds und/ oder des Hochfrequenzmagnetfeldes ermittelt, um so eine Phasenverschiebung zwischen dem Niederfrequenzmagnetfeld und dem Antwortmagnetfeld bzw. zwischen dem Hochfrequenzmagnetfeld und dem Antwortmagnetfeld zu ermitteln. Aus Gründen der Reproduzierbarkeit ist die Vorrichtung vorteilhaft so eingerichtet, dass der Vorrichtung die Phasenlagen von modulierendem Magnetfeld und Hochfrequenzmagnetfeld bekannt sind und zwar wenigstens zu Beginn einer Datenerfassung.

Vorteilhaft findet die Erfassung von Messdaten gemäß einer Abtastfrequenz statt. Beträgt die Abtastfrequenz also beispielsweise 1000 Hz, dann werden pro Sekunde 1000 Messdaten erfasst.

In einer bevorzugten Ausführungsform der Erfindung ist die Aufnahmefrequenz des Analog-Digital-Wandlers ein ganzzahliges Vielfaches der Frequenz des Hochfrequenzmagnetfeldes. Beträgt beispielsweise die Frequenz des Hochfrequenzfelds 50 kHz, dann werden Messwerte mit einer Frequenz von 100 kHz, 150 kHz oder 200 kHz erfasst. Dadurch ist sichergestellt, dass auch das Aufnehmen der Datenpunkte während eines Messvorgang zeitlich konstant zu den magnetischen Feldern passiert. Ist diese Aufnahme zusätzlich mit der Erzeugung getriggert, so ist sichergestellt, dass bei verschiedenen Messvorgängen immer zum gleichen Zeitpunkt und gleich verteilt über mehrere Perioden der magnetischen Frequenzen die Daten aufgenommen werden. Weiterhin bevorzugt werden die Frequenzgeneratoren und der Demodulator/der ADC vom gleichen Quarz mit einem Grundtakt, z.B. 50 MHz, versorgt. Dies sorgt dafür, dass bei einer Grundtaktänderung, z.B durch Erwärmung des Quarzes, eine identische Änderung bei allen Komponenten auftritt.

In einer bevorzugten Ausführungsform der Erfindung ist die Messeinrichtung so eingerichtet, dass diese sowohl eine Phasenverschiebung zwischen zumindest einer Phase eines angelegten magnetischen Wechselfelds und einer Phase eines Antwortmagnetfelds ermitteln kann, als auch ein Verhältnis von Harmonischen, die das Antwortmagnetfeld umfasst. Wenn diese Informationen über die Amplituden und Phasen von mehreren Frequenzmischkomponenten einer Probe mit darin enthaltenen magnetischen Partikeln ermittelt werden, so können verschiedene Arten von magnetischen Partikeln besonders zuverlässig bestimmt werden. Verschiedene Arten von Teilchen weisen unterschiedliche Oberwellenspektren, also Spektren von Harmonischen, auf. Zum Beispiel ist es basierend auf einer Partial - Least - Squares (PLS) -Analyse der Antwortspektren einer Probe mit einer unbekannten Mischung mehrerer Arten von Partikeln mit variablen Mengen möglich, die Zusammensetzung der Probe zu extrahieren. Dies gelingt beispielsweise, indem der PLS-Algorithmus zuvor mithilfe von bekannten Mischungen bzw. Zusammensetzungen angelernt wird. Auf diese Weise können aus einer PLS-Analyse die Arten aller Bestandteile an magnetischen Partikeln einer Probe ermittelt werden. Es ist außerdem möglich, auch die Menge an Partikeln einer jeden Art zu bestimmen. Dafür werden entsprechende Amplitudenhöhen erfasst und ausgewertet. Alternativ können ein neuronales Netzwerk oder andere Maschinen-Lern-Algorithmen mit Proben bekannter Zusammensetzung trainiert und zur Analyse unbekannter Proben verwendet werden.

In einer Probe, bei der verschiedene Arten von magnetischen Partikeln mit unterschiedlichen Antikörpern oder Aptameren oder ähnlichen Molekülen gegen verschiedene Analyten funktionalisiert wurden, erlaubt die Unterscheidung der verschiedenen Partikelarten somit eine Unterscheidung der verschiedenen Analyten in einer Probe und somit einen sogenannten multiparametrischen Immunoassay. Natürlich kann dieses Prinzip auch in anderen Bereichen wie der Medizin, der Umwelttechnologie oder in ähnlichen Anwendungsfeldern verwendet werden, wenn (verschiedene) magnetische Partikel selbst die Substanzen sind, die nachgewiesen werden sollen, oder wenn magnetische Partikel an die gesuchten Ziel-Substanzen gebunden werden oder auf andere Weise eine Verbindung zwischen ihnen hergestellt wird, zum Beispiel durch Einlagern der magnetischen Partikel in die Ziel-Substanz.

Im Falle von sehr starken externen Magnetfeldern können magnetische Partikel vollständig magnetisch gesättigt sein. Messsignale können dann sehr schwach sein, selbst wenn eine hohe Anzahl an magnetischen Partikeln vorhanden ist. Um derartige Störungen zu erkennen, verfügt die Vorrichtung in einer Ausgestaltung der Erfindung über ein Messsystem beispielsweise umfassend ein Magnetometer, mit dem starke externe Magnetfelder erkannt werden. Bei dieser Ausgestaltung gibt es ein visuelles und/oder akustisches Anzeigesystem für eine Anzeige der Störung insbesondere bei Überschreiten eines vorgegebenen Schwellwertes. Alternativ oder ergänzend gibt es eine Steuerungseinrichtung, die das Messen in Abhängigkeit von einer solchen Störung steuert. So kann beispielsweise durch die Steuerungseinrichtung die Durchführung einer Messung verhindert werden, wenn ein übermäßig starkes externes Magnetfeld detektiert wird oder bei genügend schwachen externen Magnetfeldern eine entsprechende Korrektur durchführt.

Die Vorrichtung umfasst in einer Ausgestaltung eine Einrichtung zur Erzeugung eines statischen Magnetfeldes. Eine Einrichtung zur Erzeugung eines statischen Magnetfeldes liegt vor, wenn die Vorrichtung so beschaffen ist, dass nicht nur die zwei magnetischen Wechselfelder erzeugt werden können, sondern außerdem zusätzlich ein statisches Magnetfeld. Dafür kann beispielsweise ein Permanentmagnet oder eine Spule genügen. Es sind je nach Stärke eines statischen Magnetfeldes sowohl gerade als auch ungerade Harmonische messbar, wodurch weiter verbessert magnetische Partikel bestimmt werden können. Die Signalausprägung, z.B. die Amplitudenhöhe jeder Mischfrequenz, ist dabei zusätzlich abhängig von der Stärke des statischen Magnetfelds. Die Einrichtung zur Erzeugung eines statischen Magnetfeldes kann ferner Teil des Messsystems sein, mit dem übermäßig starke externe Magnetfelder erkannt werden können. Durch Einschalten eines statischen Magnetfeldes werden Amplituden von Harmonischen geändert. Ein solches Einschalten kann also dazu genutzt werden, um zu prüfen, ob Amplitudenänderungen in erwarteter Größenordnung auftreten. Ist dies nicht der Fall, so kann auf das Vorhandensein von externen starken Magnetfeldern geschlossen werden, die eine Messung und damit eine Bestimmung von magnetischen Partikeln übermäßig stören. Auch die Betrachtung von Verhältnissen zwischen geraden und ungeraden Harmonischen kann dazu genutzt werden, um externe Störungen zu erkennen und geeignet darauf zu reagieren, beispielsweise durch Ausgabe eines Warnsignal oder aber gesteuert durch eine Steuereinrichtung durch Unterbrechung einer Messung.

Die Erzeugung von zwei magnetischen Wechselfeldern mit zwei unterschiedlichen Frequenzen kann in einer Ausgestaltung der Erfindung durch Kombination zweier einzelner Frequenzquellen über einen Addierer oder durch Verwendung einer programmierbaren Quelle erfolgen, die nicht nur ein reines Sinussignal liefert, z.B. ein Arbiträrsignal-Generator. Darüber hinaus kann diese Vorrichtung so eingerichtet sein, dass zusätzlich ein statisches Magnetfeld erzeugt werden kann und zwar beispielsweise mithilfe derselben Spule, die auch der Erzeugung eines magnetischen Wechselfeldes dient.

Es können zwei getrennte Spulen für Referenz und Probenmessung vorhanden sein, ggf. mit eigener Signalerzeugung und Signalverstärkung (Anregung/Detektion). Zusätzliche Schirmungsspulen oder Balancierungsspulen oder nicht rotationssymmetrische Spulen oder nicht zylindrische Spulen können vorhanden sein.

Die magnetischen Wechselfelder müssen nicht sinusförmig sein. Neben der Verwendung reiner sinusförmiger magnetischer Wechselfelder können auch andere Wellenformen zur Anregung der magnetischen Partikel verwendet werden. Anders geformte magnetische Wechselfelder können nützlich sein, um zusätzliche Informationen zu erhalten, umso weiter verbessert magnetische Partikel bestimmen zu können.

Die magnetischen Wechselfelder können z.B. mit Frequenz multiplizierenden Teilen aus einer gemeinsamen Frequenz erzeugt werden. Dies kann z.B. sowohl eine höhere als auch eine niedrigere Grundfrequenz sein, z.B. mit Hilfe eines Frequenzteilers. Weiterhin ist es z.B. möglich, diese Frequenz unabhängig oder bevorzugt abhängig/synchronisiert zu erzeugen, z.B. mithilfe von Direct Digital Synthesis (DDS)-Chips. Alternativ können diese auch direkt von einer gemeinsamen Frequenzquelle ausgegeben werden, zum Beispiel mithilfe eines FPGA, welcher zwei Digital-zu-Analog-Wandler steuert.

Die Erfassung der Amplituden der beiden Frequenzen f1 und f2 während des gesamten Messverlaufs im Signal der Detektionsspule ist in einer Ausgestaltung der Erfindung ein Kriterium für die Stabilität und für die Erkennung möglicher Fehler im Detektionssystem. Anregung Amplitudenpegel werden also überwacht und in Abhängigkeit davon wird das Messen gesteuert und/oder es werden Warnsignale bei Überschreiten von Grenzwerten ausgegeben. Zusätzlich/Alternativ kann der Gesamtpegel des Spulensignals, z.B. der Peak-Peak-Wert, als weiteres Stabilitätskriterium bzw. Kriterium zur Erkennung von Fehlern verwendet werden. Als Beispiel sei hier aufgeführt, das ein externes starkes elektrisches Feld so stark in die Elektronik einstrahlt, dass z.B. die Verstärker an ihre Grenzen geführt werden und damit dann auch die anderen Signale nicht ordentlich verstärkt werden können. Dieses Feld muss dabei nicht im Bereich der zu detektierenden Mischfrequenzen liegen, hat aber dennoch eine starke Auswirkung auf das Gesamtsystem.

In einer Ausgestaltung der Erfindung erfolgt eine Detektion des Antwortmagnetfeldes über einen längeren Zeitraum ohne Probe zu Prüf- und Kontrollzwecken. Veränderungen am Messaufbau oder an der Umgebung können so ermittelt werden, da diese anhand des Antwortmagnetfeldes erkennbar sein können. Dies kann nützlich sein als Kriterium für die Qualität einer Messung und kann Hinweise geben für ggf. nötiges Nachkalibrieren bzw. der Erkennung von Fehlern im Messsystem. Zusätzlich oder alternativ kann dies auch (automatisiert/manuell) wiederholt zwischen den Messungen erfolgen. Damit kann die Stabilität kontrolliert werden bzw. z.B. genauere Differenzmessungen (z.B. Probensignal - Hintergrundsignal) ermöglicht werden.

Weiterhin ermöglicht die Erfassung von weiteren Frequenzen (auch von solchen, die nicht zum Bereich der Anregungsfrequenzen bzw. Mischfrequenzen gehören) die Ermittlung von Umweltstörungen auf Basis von elektromagnetischen Feldern. Hierbei gehören zum Beispiel Felder, die durch die Spannungsversorgung kommen bzw. von Spannungsleitungen und anderen AC Magnetfeld Quellen erzeugt werden (u.a. Netzrauschen, z.B. 50 Hz und Harmonische in Deutschland, und andere Frequenzen im Falle von anderen Netzen bzw. bei Einsatz von DC-AC-Wandlern). Besonders nützlich ist die Detektion von Störungen, die die gleiche oder eine ähnliche Frequenz, oder eine Harmonische der zur Anregung und Detektion verwendeten Frequenzen aufweisen, damit diese Störungen nicht als Messsignale fehlinterpretiert werden, sondern auf die Umgebung zurückgeführt werden. Solche Messungen erfolgen in einer Ausgestaltung der Erfindung sowohl mit als auch ohne Probe, um einen möglichst störungssicheren Betrieb zu ermöglichen. Hierbei ist es möglich, aber nicht darauf beschränkt, dass in einem solchen Fall dem Anwender eine Warnung angezeigt wird oder dass die Messung nicht durchgeführt wird oder dass diese Störungen berücksichtigt werden und korrigierte Werte ausgegeben werden, ggf. mit zusätzlicher Anpassung der Amplituden und Betrachtung des Effekts auf das detektierte Signal. Weiterhein ist es auch möglich, im Falle der Detektion von Störfrequenzen einen automatisierten intelligenten Wechsel zu Frequenzen, die nicht gestört sind, herbeizuführen.

Wie beschrieben, muss das auslösende Signal nicht von der Signalquelle erzeugt werden. Es ist auch möglich, es direkt aus dem Signal zu generieren, und es muss auch kein "digitales" Signal mit nur zwei Zuständen sein. Hierbei geht es um die Art zu triggern. Es ist möglich, dass die Signalquellen selbst ein Trigger-Signal ausgeben, wie es z.B. manche DDS-Chips machen. Oder es kann ein geeignetes, quasi zweizuständiges Triggersignal mittels weiterer Komponenten wie zum Beispiel eines Operationsverstärkers in Schmitt-Trigger-Konfiguration erzeugt werden. Weiterhin ist es auch möglich, dass ein Signal mehr als nur zwei Zustände hat, wie zum Beispiel eine Sinuswelle, bei der beispielsweise das Über-/Unterschreiten eines bestimmten Schwellenwerts als Trigger dient.

Nachfolgend wird die Erfindung anhand von Ausführungsbeispielen näher erläutert. Es zeigen
- Fig. 1:: Anregungs- und Antwortfrequenzen
- Fig. 2:: Ausführungsbeispiel mit zwei ADCs
- Fig. 3:: Ausführungsbeispiel mit Auslöseeinrichtung
- Fig. 4:: Ausführungsbeispiel mit Auslöseeinrichtung
- Fig. 5:: Ausführungsbeispiel mit statischem Magnetfeld
- Fig. 6:: Messwerte
- Fig. 7:: Aufbau eines Ausführungsbeispiels

Die Figur 1 zeigt ein Beispiel für ein Hochfrequenzmagnetfeld mit einer Frequenz f1 und einem Niederfrequenzmagnetfeld mit einer Frequenz f2 und eine daraus resultierende Harmonische mit einer Frequenz f1 + 2·f2. Gezeigt wird die Amplitude A über die Zeit T. Hierbei wurde zur besseren grafischen Darstellung, wie zuvor erwähnt, der Faktor f1/f2 sehr gering gewählt. Bevorzugt hat er in einer realen Messsituation einen größeren Wert von mindestens 100.

Die in den Figuren 2 bis 5 schematisch gezeigten Beispiele beziehen sich auf Realisierungen, die es erlauben, Phasenverschiebungen zwischen Niederfrequenzmagnetfeld und Antwortmagnetfeld oder Hochfrequenzmagnetfeld und Antwortmagnetfeld zu ermitteln. Die Reihenfolge der Spulen und die Art der Spulen sind nur beispielhaft dargestellt. Sie ist nicht auf diese Reihenfolge oder Art beschränkt. Nicht gezeigt sind zusätzlich benötigte Komponenten wie Leistungsverstärker, Filter, Operationsverstärker usw.

Wird ein Signal eines Antwortmagnetfeldes mit einem Analog-Digital-Wandler (ADC) erfasst, können die Amplituden der Harmonischen und relative Phasendifferenzen zwischen Harmonischen extrahiert werden. Nachfolgend werden vorteilhafte Ausführungsformen beschrieben, die es darüber hinaus ermöglichen, auch Phasendifferenzen zwischen Niederfrequenzmagnetfeld und Harmonischen oder Hochfrequenzmagnetfeld und Harmonischen zu ermitteln.

Die Figur 2 zeigt zwei Wechselstromquellen 1 und 2. Eine Hochfrequenzanregung mit einer Frequenz f1 erfolgt durch die Wechselstromquelle 1. Die Wechselstromquelle 1 dient also der Erzeugung eines Hochfrequenzmagnetfeldes. Eine Niederfrequenzanregung mit einer Frequenz f2 erfolgt durch die Wechselstromquelle 2. Die Wechselstromquelle 2 dient also der Erzeugung eines Niederfrequenzmagnetfelds.

Es ist eine Synchronisierungseinrichtung 3 vorhanden, die die beiden Wechselstromquellen 1 und 2 miteinander synchronisiert. Durch die Synchronisierung ist während des Betriebs eine Phasenbeziehung zwischen dem Hochfrequenzmagnetfeld und dem Niederfrequenzmagnetfeld der Vorrichtung bekannt.

Zur Erzeugung des Hochfrequenzmagnetfeldes ist die Wechselstromquelle 1 mit einer inneren Spule 4 elektrisch verbunden. Zur Erzeugung des Niederfrequenzmagnetfeldes ist die Wechselstromquelle 2 mit einer äußeren Spule 5 elektrisch verbunden. Die innere Spule 4 ist innerhalb der äußeren Spule 5 angeordnet. Werden elektrische Ströme durch die Wechselstromquellen 1 und 2 an die entsprechenden Spulen 4 und 5 geliefert, so werden entsprechende magnetische Wechselfelder mit hoher Frequenz *f*₁ und niedriger Frequenz *f*₂ erzeugt. Das Signal einer nicht gezeigten Probe wird von einer Detektionsspule 6 erfasst. Die Detektionsspule 6 besteht bevorzugt wie dargestellt aus zwei Einzelspulen, die eine differenzielle Spule bilden, da so sehr leicht in der Praxis direkt induzierte Signale unterdrückt werden können. Eine Spule ist also rechts und die andere links herum gewickelt. Eine Detektionsspule kann aber auch eine einzelne Spule sein. Eine jede Spule kann aus mehreren Komponenten bestehen.

Es ist also auch möglich, dass die Detektionsspule aus mehreren Komponenten besteht, so z.B. eine differenzielle Spule ist, bei der die Referenzsspule über mehrere Bereiche aufgeteilt ist, um so Inhomogenitäten/Inbalancen in den Anregespulen, mit denen das Hochfrequenzmagnetfeld und das Niederfrequenzmagnetfeld erzeugt werden, besser zu unterdrücken z.B. ½^{∗}Referenzspule, Detektionsspule, ½^{∗}Referenzsspule. Das Signal umfasst eine Harmonische mit einer Frequenz f3, wobei f3 beispielsweise f1 + 2·f2 sein kann. Das erfasste Signal wird ggfs. vorbereitet und zu einem Analog-Digital-Wandler 7 weitergeleitet. Der Analog-Digital-Wandler 7 digitalisiert das analoge Signal. Ein weiterer Analog-Digital-Wandler 8 digitalisiert das Wechselstromsignal der Wechselstromquelle 2. Aus den Werten, die durch die beiden Analog-Digital-Wandler 7 und 8 digitalisiert wurden, kann nun die Phasendifferenz zwischen der Harmonischen mit der Frequenz f3 und der Wechselstromquelle 2 mit der Frequenz f2 ermittelt werden. Da die beiden Wechselstromquellen 1 und 2 miteinander synchronisiert sind, kann auch die Phasendifferenz zwischen der Harmonischen mit der Frequenz f3 und der Wechselstromquelle 1 mit der Frequenz f1 ermittelt werden beziehungsweise durch Kenntnis der Phasenlage von f1 und f2 bzw. f2 und dem Phasenversatz zu f1 eine virtuelle Referenzphasenlage von f3 berechnet werden. Damit ist es dann möglich, direkt den Phasenversatz von f3 zwischen Anregung und Detektion zu bestimmen.

Das Ausführungsbeispiel gemäß Figur 2 ermöglicht die Bestimmung von magnetischen Partikeln unabhängig von der Art der synchronisierten Frequenzquelle, die zur Erzeugung von hohen und niedrigen Frequenzen verwendet wird. Die Messung kann zu jedem Zeitpunkt des Signals f2 beginnen.

Es werden zwei Analog - Digital - Wandler 7 und 8 benötigt. Es werden entsprechend viele Daten erfasst und verarbeitet. Ein entsprechend hoher Rechenaufwand ist erforderlich, beispielsweise zur Durchführung von zwei Fouriertransformationen, um eine virtuelle Referenzphase für f3 zu erstellen.

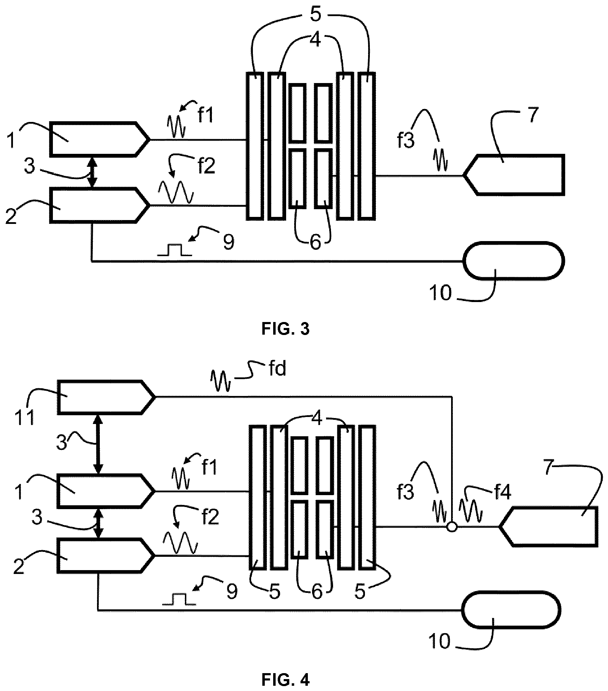

Die Figur 3 zeigt eine Abwandlung der in der Figur 1 gezeigten Ausführungsform. Bei der Ausführungsform der Figur 3 wird kein zweiter Analog-Digital-Wandler 8 eingesetzt. Stattdessen wird ein digitales Triggersignal 9, welches von Wechselstromquelle 2 bei jeder Periode von f2 ausgegeben wird, verwendet, um durch eine Auslöseeinrichtung 10 die Durchführung einer Bestimmung von magnetischen Partikeln auszulösen. So kann eine Erfassung von Messwerten durch die Auslöseeinrichtung 10 gestartet werden, um so die Bestimmung von magnetischen Partikeln auszulösen. Beispielsweise wird das Triggersignal 9 verwendet, um eine Wandlung von analogen Messwerten in digitale Daten durch den Analog-Digital-Wandler 7 zu starten, um so die Bestimmung von magnetischen Partikeln auszulösen. Es kann aber auch auf andere Weise die Messung oder die Aufzeichnung von Messwerten gestartet werden. Es gelingt so, eine Synchronisierung der erfassten digitalen Daten mit der Phase des Niederfrequenzmagnetfeldes, das mit der Frequenz f2 schwingt. Es wird dadurch möglich, eine Phasenverschiebung zwischen Harmonischen und dem Niederfrequenzmagnetfeld zu ermitteln. Da das Niederfrequenzmagnetfeld mit dem Hochfrequenzmagnetfeld synchronisiert ist, kann auch eine Phasenverschiebung zwischen Harmonischen und dem Hochfrequenzmagnetfeld ermittelt werden, beziehungsweise in Bezug auf die virtuelle Referenzphase der Harmonischen.

Es wird bei dieser Ausführungsform nur ein Analog-Digital-Wandler 7 benötigt. Ein Auslösen gelingt auf technisch sehr einfache Weise, so zum Beispiel durch Verwendung eines FPGA und auch unabhängig von der Art der synchronisierten Wechselstromquellen 1, 2, die zur Erzeugung von hohen und niedrigen Frequenzen verwendet werden.

Es wird bei dieser Ausgestaltung eine Frequenzquelle oder externe Hardware benötigt, die ein Triggersignal 9 liefert. Dies kann zum Beispiel entweder von der Wechselstromquelle 2 stammen oder zum Beispiel aus einer analogen Niederfrequenzsignalkomponente, welches z.B. mit einem Operationsverstärker in einer Schmitt-Trigger-Konfiguration erhalten werden kann.

Ein spezifischer Triggerpunkt eines Frequenzsignals der anregenden Magnetfelder (f1 oder f2), also des Niederfrequenzsignals im Fall der Figur 3, wie z.B. ein Nulldurchgang, muss abgewartet werden beziehungsweise passieren, ehe mit einer Bestimmung von magnetischen Partikeln begonnen werden kann. Nach Start der Messprozedur wird beginnend mit dem nächsten Triggersignal eine spezifische Anzahl von Datenpunkten vom Analog-Digital-Wandler aufgenommen, d.h, der Start der Messperiode wird durch den Trigger festgelegt.

Aufgrund der einfacheren Realisierbarkeit ist dies die bevorzugte Messsituation, allerdings ist es auch möglich, dass die Signale vom Analog-Digital Wandler kontinuierlich gewandelt und zwischengespeichert werden, und bei einem spezifischen Triggersignal die zuvor aufgenommenen Daten verwendet werden (Pre-Trigger). Kombinationen dieser Verfahren sind auch möglich. Hierbei ist es zu bevorzugen, dass die Frequenzen der Analog-zu-Digital-Wandlung und die der Erzeugung der Niederfrequenz f2 synchronisiert sind und bevorzugt mit einer festgelegten Startphasenlage erfolgen.

Die Figur 4 zeigt eine im Vergleich zur Figur 3 weiter entwickelte Vorrichtung mit einer Signalquelle 11, die ein Signal mit einer Demodulationsfrequenz fd ausgibt. Hierbei ist es wiederum vorteilhaft, wenn fd ein ganzzahliges Vielfaches der Niederfrequenz ist, da so die Phasendifferenz für alle Mischkomponenten leicht ermittelt werden kann bzw. konstant ist. Die Signalquelle ist grundsätzlich eine Wechselstromquelle. Die Phase der Signalquelle 11 wird mit der Phase der Wechselstromquelle 1 vorteilhaft durch eine Synchronisierungseinrichtung 3 synchronisiert. Ein Signal mit der Frequenz f3, das von der Detektionsspule 6 aufgenommen wird, wird zuerst mit dem Signal gemischt, das durch die Signalquelle 11 für ein Heruntermischen erzeugt worden ist. Es entsteht so ein demoduliertes Signal mit einer im Vergleich zur Frequenz f3 verringerten Frequenz f4. Danach wird das heruntergemischte Signal mit der verringerten Frequenz f4 durch den Analog-Digital-Wandler 7 digitalisiert. Wiederum wird vorteilhaft mit f2 getriggert, um eine feste/bekannte Startphasenlage zu erhalten.

Im Fall der Figur 4 wird demnach die Verwendung einer digitalen Demodulation mit einer analogen Demodulation kombiniert. Ein analoger Demodulationsteil ist, wie in der Figur 4 gezeigt, vor dem Analog-Digital-Wandler 7 angeordnet, um als (erste) Demodulationsstufe zu dienen. Durch diese Demodulationsstufe wird der Signalfrequenzbereich in einen Zwischenfrequenzbereich verschoben. In einer zweiten Stufe kann die Demodulation der gewünschten Frequenzmischkomponenten auf digitale Weise durchgeführt werden. Mit dieser Kombination ist es möglich, einen Analog-Digital-Wandler 7 zu verwenden, der eine niedrige Abtastfrequenz hat. Dies führt dazu, dass beispielsweise bei einer Fouriertransformation weniger Abtastungen pro Zeitintervall benötigt werden, um die gleiche Frequenzauflösung zu erhalten. Die niedrigere Abtastrate bedeutet, dass die Auswahl von Analog-Digital-Wandlern weniger anspruchsvoll wird und Analog-Digital-Wandler mit besseren Eigenschaften verwendet werden können. Wenn weniger Abtastwerte benötigt werden, sind zusätzlich die folgenden Vorteile relevant: Weniger Rechenaufwand ist für die Demodulation erforderlich, es wird weniger Speicherplatz benötigt, um gemessene Daten kurz oder lang vor oder nach einer Signalanalyse zu speichern (Rohdaten oder berechnete Daten), und es können langsamere Komponenten zum Aufnehmen und Übertragen von Signalen verwendet werden. So kann ein Detektionsteil vereinfacht als Spulensignal -> Analoge Demodulation -> Analog-Digital-Wandler beschrieben werden. Die analoge Demodulation kann abhängig vom Zweck der Messung mit verschiedenen Frequenzen durchgeführt werden. Vorteilhafte Beispiele dafür sind:
(1) Die analoge Demodulationsfrequenz fd ist in einer Ausgestaltung gleich der hohen Frequenz f1 gewählt. In diesem Fall sind alle Oberwellen bzw. Harmonischen mit einer Addition erfassbar (*f*₁ + 1·*f*₂, *f*₁ + 2·*f*₂, ... allgemein: f1 + n·f2 mit jeder natürlichen Zahl n). Es ist dann aber nicht möglich, Informationen über Oberwellen mit einem Minuszeichen zu erhalten wie f1 - 1·f2, f1 - 2·f2, ... oder allgemein f1 - n·f2 mit einer beliebigen natürlichen Zahl n. Es werden nach der analogen Demodulation mit f1 die Oberwellen f1 + n·f2 nicht mehr auf der Frequenz f1 + n·f2 liegen, sondern auf die Frequenz n·f2 herunterkonvertiert sein.
(2) Beim vorgenannten Beispiel sind die Oberwellen f1 - n·f2 bei der Verwendung von f1 zur Demodulation nicht mehr analysierbar. Wenn solche Oberwellen ebenfalls von Interesse sind, wird eine kleinere Frequenz fd zur Demodulation verwendet. Im Allgemeinen werden die Harmonischen dann bei neuen Frequenzen gemäß dem folgenden Schema gefunden: Die Frequenz der n-ten Harmonischen ist gleich f1 ± n·f2 - fd. Bei dieser Ausgestaltung der Erfindung ist die obere Grenze für detektierbare Frequenzen durch die Nyquist-Grenze gegeben: f1 + n·f2 - fd ≤ Frequenz der Nyquist-Grenze der ADC-Abtastung. Wie zu sehen ist, ist die obere Frequenzgrenze immer noch durch eine Nyquist-Grenze gegeben, aber jetzt sind die Frequenzen kleiner geworden. Während die untere Grenze für detektierbare Harmonische f1 - n·f2 - fd ≥ 0 Hz ist, ist das gesamte Frequenzband 0 Hz ≤ Frequenz ≤ Nyquist-Grenze der ADC-Abtastung. Wie zu sehen ist, ist (1) ein Spezialfall von (2). Im Folgenden wird ein Beispiel gegeben. Sei f2 = 50 Hz, f1 = 30 kHz = f2 · 600 und fd = 29000 kHz = f2 · 580. Die untere Grenze für die betragsmäßig größte zu bestimmende Harmonische ist gegeben als 30 kHz - 29 kHz - n · 50 Hz = 0, also 1000 Hz / 50 Hz = n, entsprechend n = 20.

Zum Beispiel kann mit einem Analog-Digital-Wandler 7 mit einer Abtastfrequenz von mindestens 4 kSps ein symmetrischer Bereich von Harmonischen zwischen f1 - 20 · f2 bis f1 + 20 · f2 analysiert werden.

Mit dieser Kombination von analoger und digitaler Demodulation kann immer noch die Charakteristik vieler Frequenzkomponenten gleichzeitig analysiert werden. Der erforderliche Rechenaufwand und die Zahl benötigter Datenpunkte sind vorteilhaft geringer. Auch die benötigte Verarbeitungsgeschwindigkeit ist vorteilhaft geringer.

Um vorteilhaft eine stabile Phasenbeziehung zu erhalten, wird die für die analoge Demodulation vorgesehene Frequenzquelle 11 mit der Wechselstromquelle 1 durch eine Synchronisierungseinrichtung 3 synchronisiert. Es werden dann mehr Informationen erhalten, um weiter verbessert qualitativ bestimmen zu können.

Vorteilhaft wird fd als ganzzahliges Vielfaches der Niederfrequenz f2 gewählt, damit die Phasenlage/Phasendifferenz konstant/leicht zu ermitteln ist.

Wird ein Analog-Digital-Wandler eingesetzt, der durch ein Triggersignal wie in der Figur 3 gezeigt gestartet wird, um Daten zu digitalisieren, so kann es konstruktionsbedingt im Falle von permanent laufender Analog-Digital-Wandlung einen (variablen) Versatz zwischen ermittelter und tatsächlicher Phasenverschiebung geben. Um einen solchen Versatz zu korrigieren, ist es vorteilhaft, wie anhand der Figur 3 offenbart den Analog-Digital-Wandler zu triggern, um beispielsweise den Zeitpunkt der Verarbeitung durch den Analog-Digital-Wandler nebst Weiterverarbeitung auf den Anfang der Niederfrequenz zu legen. Zugleich wird mittels eines parallel aufgenommenen Anregungssignals bzw. Referenzsignals an einem zweiten Analog-Digital-Wandler, wie dieser in der Figur 2 gezeigt wird, die genaue Phasenlage ermittelt. Eine solche Vorrichtung umfasst dann also sowohl einen Aufbau wie in der Figur 2 gezeigt und zusätzlich eine Auslöseeinrichtung 10 gemäß der Figur 3. Insgesamt gelingt es, die Phasenverschiebungen zu ermitteln und so magnetische Partikel zu bestimmen.

Beim vorgenannten Ausführungsbeispiel ist es von Vorteil, anstatt der Aufnahme der Niederfrequenz direkt eine passende höhere Frequenz wie zum Beispiel die verwendete Hochfrequenz f1 aufzunehmen. Dies bietet den Vorteil, dass gesuchte Phasenverschiebungen genauer bestimmt werden können.

In einem weiteren Ausführungsbeispiel ist die Vorrichtung so eingerichtet, dass eine Signalaufnahme durch einen Analog-Digital-Wandler 7 bereits erfolgt, bevor das Niederfrequenzmagnetfeld und/oder das Hochfrequenzmagnetfeld und/oder eine zusätzliche Information (z.B. Peak) eingeschaltet werden. Sobald ein entsprechendes Antwortmagnetfeld/Signal gemessen wird, wird dieser Zeitpunkt verwendet, um daraus eine Phasenverschiebung zwischen Niederfrequenzmagnetfeld und/oder Hochfrequenzmagnetfeld einerseits und dem Antwortmagnetfeld andererseits zu ermitteln.

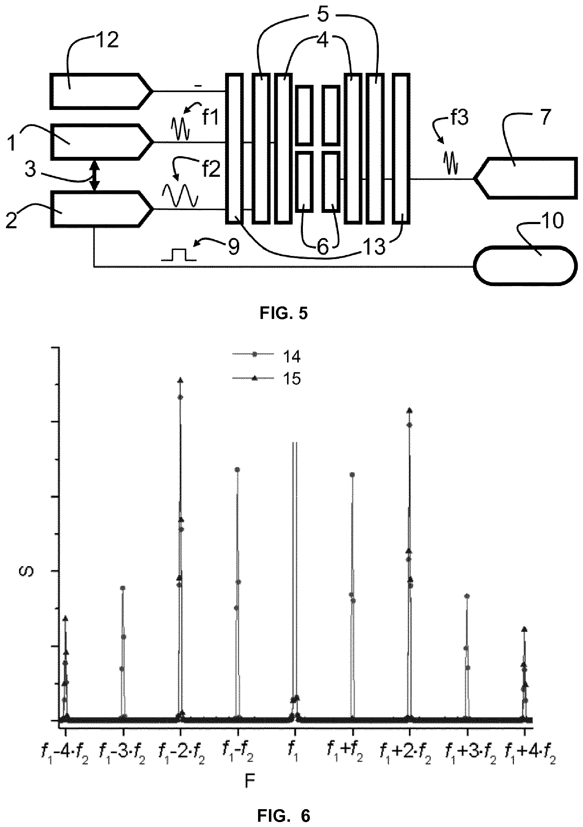

Die Figur 5 zeigt ein Ausführungsbeispiel mit einer Gleichstromquelle 12 und einer Spule 13, um ein statisches Magnetfeld (statisches magnetisches Offsetfeld) zu erzeugen. Die beiden Spulen 4 und 5 sind innerhalb der Spule 13 angeordnet.

Das statische magnetische Offsetfeld, dem die magnetischen Partikel zusätzlich ausgesetzt werden, führt zum Auftreten von zusätzlichen geraden harmonischen Frequenzmischtermen, zusätzlich zu den eingangs genannten ungeraden. Figur 6 zeigt gemessene Frequenzmischkomponenten aus einer Probe magnetischer Nanopartikel, gemessen mit statischem magnetischem Offsetfeld (siehe Kurven 14) und ohne statisches magnetisches Offsetfeld (siehe Kurven 15). Es ist das Messsignal S gegenüber der Frequenz F aufgetragen. Es ist deutlich zu sehen, dass die Mischterme f1 ± 2 · f2 und f1 ± 4 · f2 in beiden Fällen auftreten, während die Mischterme f1 ± 1 · f2 und f1 ± 3 · f2 nur im Fall eines statischen magnetischen Offsetfeldes auftreten.

Es stehen damit mehr oder zumindest andere Informationen zur Bestimmung von magnetischen Partikeln zur Verfügung, wenn statisches magnetisches Offsetfeld hinzukommt. Magnetische Partikel können daher verbessert bestimmt werden.

In Abhängigkeit von dem Wert des magnetischen Offsetfeldes ändern sich die (absoluten und relativen) Werte der Amplituden der Frequenzmischterme. Dies wird in einer vorteilhaften Ausgestaltung berücksichtigt, so zum Beispiel rechnerisch, wenn aus der Amplitudenhöhe der Mischterme auf die in der Probe vorhandenen Mengen unterschiedlicher magnetischer Partikel geschlossen werden soll.

Da ein Vorhandenes statisches Magnetfeld die Höhe einer Amplitude verändert, werden in einer vorteilhaften Ausgestaltung Amplituden von verschiedenen Mischfrequenzen durch eine Detektionseinrichtung überwacht. Verändert sich eine Amplitude unplanmäßig, so signalisiert dies einen störenden Einfluss durch ein unplanmäßig auftretendes (statisches) Magnetfeld. In einer Ausgestaltung gibt es eine Warneinrichtung, die einen Benutzer warnt, wenn durch eine detektierte unplanmäßige Amplitudenänderung ein unplanmäßig vorhandenes statisches Magnetfeld erkannt wird. Alternativ oder ergänzend verhindert die Detektionseinrichtung mithilfe einer Steuereinrichtung eine Messung, um verfälschte Ergebnisse zu vermeiden. Es ist also bei dieser Ausgestaltung möglich, das Vorhandensein eines statischen Magnetfeldes auch ohne Verwendung eines separaten Magnetometers zu detektieren. Dies kann auch mithilfe einer Referenzprobe geschehen, deren spezifisches Signalverhalten in Abhängigkeit vom Magnetfeld bekannt ist und somit ggf. genauere Rückschlüsse auf die Umgebung gezogen werden können.

Alternativ kann auf der Basis eines mathematischen Modells, das die Signalveränderung aufgrund des Vorhandenseins eines magnetischen Offsetfeldes berücksichtigt, auf die Menge magnetischer Nanopartikel geschlossen werden, was es ermöglicht, trotz sich veränderten externem Offsetfeld eine stabile und zuverlässige Messung zu erzielen. Natürlich ist es auch möglich, dieses Verhalten bei einem statischen Offsetfeld gezielt zu benutzen. Mithilfe der Spule 13, mit der ein statisches Magnetfeld erzeugt wird, ist es möglich, gezielt ein magnetisches Offsetfeld zu erhöhen, zu verringern oder auf gewünschte Werte einzustellen. Dadurch lassen sich die Signalstärken der verschiedenen Harmonischen gezielt beeinflussen. Die Werte bei verschiedenen magnetischen Offsetfeldern werden in einer vorteilhaften Ausgestaltung benutzt, um verschiedene magnetische Partikel identifizieren/unterscheiden zu können und/oder spezifische Eigenschaften der Partikel wie zum Beispiel bei der Herstellung den Kristallit-/Kerndurchmesser der Partikel zu ermitteln.

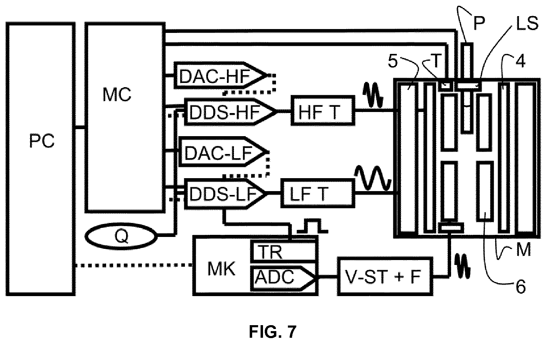

Ein Aufbau umfasst in einer vorteilhaften Ausgestaltung die folgenden in Figur 7 gezeigten Hauptkomponenten:
Ein Arduino-Mikrokontroller MC ist an einen Computer PC angeschlossen. An den Mikrokontroller MC sind 2 DDS-Chips vom Typ AD9834 zur Erzeugung der Hoch- bzw. Niederfrequenz sowie DACs vom Typ AD5620 zur Einstellung ihrer Amplituden angeschlossen. Beide DDS-Chips DDS-HF und DDS-LF werden von einem gemeinsamen Quarz Q angesteuert, um hier eine Synchronität zu gewähren. Weiterhin sind die DDS-Chips untereinander über den Mikrokontroller MC synchronisiert. Beide erzeugte Frequenzen werden dann verstärkt und über eine Leistungstreiberstufe an die jeweilige Spule 4 bzw. 5 gegeben.

Diese Stufe hat als Hauptbaustein einen Chip vom Typ BUF634. Diese Stufe gibt für jede Spule 4 und 5 das originale und das invertierte Signal aus, um so größere Spannungen an der Spule anlegen zu können (z.B. ±30V bei ±15V Versorgungsspannung, statt nur +15 V gegenüber Masse). Dieser Aufbau ist identisch für beide Anregespulen 4 und 5. Das Detektionssignal wird von der Detektionsspule 6 aufgenommen und anschließend vorgefiltert (u.a. Hochpass zum Entfernen der Anregungsfrequenzen und Tiefpass als Antialiasingfilter) und verstärkt und an einen analogen Eingang der externe Messkarte MK und zwar eine Messkarte NI6251 von National Instruments weitergeleitet. Zusätzlich ist an einem Triggereingang dieser Karte MK der Pin des DDS AD9834 angeschlossen, über den das Most Signifikant Bit (MSB) des internen DACs ausgegeben wird. Dieses Rechtecksignal wird als Trigger für den Messvorgang am PC verwendet. Dieser nimmt dann eine gewisse Anzahl an Datenpunkte, z.B. 200.000, mit einer Datenrate von 200 kSps auf und führt anschließend eine Fouriertransformation FFT durch. Ein Beispielausschnitt eines FFT Bildes ist in Fig. 6 dargestellt. Hierbei wurde an eine Extra-Spule ein variabler Gleichstrom angelegt und somit ein statisches magnetisches Offsetfeld erzeugt. In der Figur 6 sind nur die 2 Zustände mit und ohne Offsetfeld gezeigt.

Hauptkomponenten sind wie in der Figur 7 gezeigt:
- MC:: Mikrokontroller "Arduino Micro";
- DAC -HF bzw. DAC- LF:: DAC5620 von Analog Devices;
- DDS-HF bzw. DDS-LF:: AD9834 von Analog Devices;
- HF T:: Hochfrequenztreiber BUF634;
- LF T:: Niederfrequenztreiber BUF634;
- V-ST + F:: Vorverstärker+Filter: Passive Hoch und Tiefpässe;
- LS:: Lichtschranke zur Detektion, ob ein Probengefäß P eingeführt ist;
- T:: Temperatursensor zur Überwachung der Temperatur im Messkopf M;
- MK:: Messkarte: Ni6251 von National Instruments.

Zusätzlich sind noch zum Betrieb weitere Komponenten wie zum Beispiel zur Stabilisierung der Betriebsspannung, Verstärkung von Signalen, Filterung von Vorteil.

## Patentansprüche

1. Vorrichtung zur Bestimmung von magnetischen Partikeln umfassend einen Probenhalter für magnetische Partikel, zwei Magnetfelderzeugungseinrichtungen (1, 4; 2, 5) für ein Anlegen eines magnetischen Niederfrequenzwechselfeldes (f2) und eines magnetischen Hochfrequenzwechselfeldes (f1) an magnetische Partikel, die durch den Probenhalter gehalten werden können, eine Messeinrichtung, mit der eine Phasenverschiebung zwischen zumindest einer Phase eines angelegten magnetischen Wechselfelds (f1, f2) und einer Phase eines Antwortmagnetfelds (f3) ermittelt werden kann und/oder ein Verhältnis von Harmonischen, die das Antwortmagnetfeld (f3) umfasst, ermittelt werden kann, eine Auswerteeinrichtung, mit der aus einer ermittelten Phasenverschiebung und/oder aus einem ermittelten Verhältnis von Harmonischen die Art und/oder Charakteristiken und/oder Eigenschaften von magnetischen Partikeln bestimmt werden können.

2. Vorrichtung nach Anspruch 1, **dadurch gekennzeichnet, dass** diese einen Analog-Digital-Wandler (7) zur gleichzeitigen Erfassung einer Mehrzahl von Frequenzmischkomponenten umfasst.

3. Vorrichtung nach einem der vorhergehenden Ansprüche, **dadurch gekennzeichnet, dass** eine Synchronisierungseinrichtung (3) für eine Synchronisierung der beiden magnetischen Wechselfelder (f1, f2) vorhanden ist oder bereits durch ihre Funktionsweise synchronisierter Quellen.

4. Vorrichtung nach einem der vorhergehenden Ansprüche, **dadurch gekennzeichnet, dass** eine Magnetfelderzeugungseinrichtung eine Wechselstromquelle (1,2) und eine Spule (4, 5) umfasst.

5. Vorrichtung nach einem der vorhergehenden Ansprüche, **dadurch gekennzeichnet, dass** ein zweiter Analog- Digital - Wandler (8) für eine Ermittlung einer Phasenverschiebung vorhanden ist.

6. Vorrichtung nach einem der vorhergehenden Ansprüche, **dadurch gekennzeichnet, dass** eine Einheit zur numerischen Analyse der Messergebnisse vorhanden ist, mit der das vom Analog - Digital - Wandler digitalisierte Signal zur Ermittlung einer oder mehrerer Frequenzmischkomponenten transformiert wird, zum Beispiel auf Basis von Fouriertransformation oder digitaler Lock-In-Umsetzung.

7. Vorrichtung nach einem der vorhergehenden Ansprüche, **dadurch gekennzeichnet, dass** eine Auslöseeinrichtung (10) vorhanden ist, mit der ein definierter Startzeitpunkt vorgegeben oder ermittelt werden kann.

8. Vorrichtung nach dem vorhergehenden Anspruch, **dadurch gekennzeichnet, dass** eine Triggereinrichtung zur Ausgabe eines Triggersignals (9) an die Auslöseeinrichtung (10) vorhanden ist.

9. Vorrichtung nach einem der vorhergehenden Ansprüche, **dadurch gekennzeichnet, dass** einer Signalquelle (11) vorhanden ist, die das Antwortmagnetfeld derart mithilfe einer Demodulationsfrequenz (fd) modulieren kann, dass Frequenzen (f3) von Harmonischen verringert werden.

10. Vorrichtung nach dem vorhergehenden Anspruch, **dadurch gekennzeichnet, dass** die Demodulationsfrequenz (fd) so vorgesehen ist, dass diese gleich der Frequenz (f1) des Hochfrequenzwechselfeldes (f1) ist oder direkt die Quelle bzw. ein Teil der Quelle des Hochfrequenzwechselfeldes f1 verwendet wird.

11. Vorrichtung nach einem der vorhergehenden Ansprüche, **dadurch gekennzeichnet, dass** eine Signalquelle (11) mit einer Einrichtung zur Erzeugung eines der magnetischen Wechselfelder synchronisiert wird.

12. Vorrichtung nach einem der vorhergehenden Ansprüche, **dadurch gekennzeichnet, dass** diese so eingerichtet ist, dass eine Signalaufnahme durch einen Analog-Digital-Wandler (7) bereits erfolgt, bevor das Niederfrequenzmagnetfeld und/oder das Hochfrequenzmagnetfeld eingeschaltet werden.

13. Vorrichtung nach einem der vorhergehenden Ansprüche, **dadurch gekennzeichnet, dass** eine Magnetfelderzeugungseinrichtung (12, 13) zur Erzeugung eines statischen Magnetfeldes vorhanden ist.

14. Verfahren zur Bestimmung von magnetischen Partikeln mit einer Vorrichtung nach einem der vorhergehenden Ansprüche, mit den Schritten:
eine Probe mit magnetischen Partikeln wird durch den Probenhalter gehalten, mit den zwei Magnetfelderzeugungseinrichtungen (1, 4; 2, 5) wird ein magnetisches Niederfrequenzwechselfeld (f2) und ein magnetisches Hochfrequenzwechselfeld (f1) erzeugt, durch eine Messeinrichtung wird eine Phasenverschiebung zwischen zumindest einer Phase eines angelegten magnetischen Wechselfelds (f1, f2) und einer Phase einer Harmonischen des durch die Messeinrichtung gemessenen Antwortmagnetfelds (f3) gemessen und/oder ein Verhältnis von Harmonischen, die das Antwortmagnetfeld (f3) umfasst, wird gemessen, durch die Auswerteeinrichtung wird aus einer ermittelten Phasenverschiebung und/oder aus einem ermittelten Verhältnis von Harmonischen die Art von magnetischen Partikel in der Probe bestimmt.

15. Verfahren zur Charakterisierung von Partikeln und zur Unterscheidung verschiedener Partikeltypen mit einer Vorrichtung nach einem der vorhergehenden Ansprüche, **dadurch gekennzeichnet, dass** die Veränderung der Eigenschaften des Antwortmagnetfeldes aufgrund eines statischen magnetischen Offsetfelds zur Partikelanalyse verwendet wird, indem insbesondere die Veränderung der Amplituden und Phasen ihrer verschiedenen Harmonischen in Abhängigkeit von der Stärke des magnetischen Offsetfeldes registriert werden.
